# FASCICULE DE BREVET EUROPEEN

(11) **EP 1 099 426 B1**
(45) Date de publication et mention de la délivrance du brevet: **07.07.2004**
(21) Numéro de dépôt: 00403092.0
(22) Date de dépôt: 08.11.2000
(51) Int. Cl.: A61F 2/34, A61L 27/30, A61L 27/32, A61F 2/32

(54) **Cotyle de prothèse de hanche à double mobilité**
Hüftprothesengelenkpfanne mit doppelter Beweglichkeit
Hip prosthesis acetabular cup with double mobility

(30) Priorité: 09.11.1999 FR 9914044
(43) Date de publication de la demande: 16.05.2001
(73) Titulaire: Amplitude, 01700 Neyron (FR)
(72) Inventeur:
(74) Mandataire: Robert, Jean-Pierre

(56) Documents cités:
- EP-A- 0 532 439
- EP-A- 0 974 316
- WO-A-98/12994
- DE-A- 19 714 050
- FR-A- 2 615 726
- FR-A- 2 710 836
- FR-A- 2 770 769
- US-A- 5 683 399

## Description

La présente invention concerne un cotyle de prothèse de hanche à double mobilité, c'est-à-dire possédant une cupule dans laquelle peut pivoter un insert formant lui-même articulation pour la tête de la tige fémorale.

Le logement dans l'insert de la tête de la tige fémorale est rétentif, c'est-à-dire que la tête est encliquetée dans ce logement pour limiter les risques de luxation, ce qui implique, pour préserver une amplitude convenable de débattement de l'articulation, que l'insert puisse pivoter dans la cupule. Il convient alors de s'opposer à la luxation de l'insert hors de la cupule, notamment dans la direction qui présente le plus de risques. Pour ce faire, la cupule présente une surface interne de glissement qui est supérieure à l'hémisphère : elle possède une partie rétentive qui s'oppose à l'échappement de l'insert en formant une lèvre s'étendant au-delà du plan diamétral de la cupule qui contient le bord opposé à cette lèvre.

Ce cotyle connu sous le nom de cotyle de Bousquet, est fixé dans l'os iliaque au moyen de vis traversant la paroi hémisphérique du cotyle auquel s'ajoute une vis d'extérieur qui coopère avec une languette formant équerre de maintien du côté de la lèvre de rétention.

Avec ce cotyle connu il faut prévoir un cotyle gauche et un cotyle droit pour que l'implantation des vis qui le fixent soit bien orientée dans la masse osseuse qui le reçoit. En outre la présence d'orifices dans la paroi constitue une rupture de la surface de glissement de l'insert qui nuit à ce glissement en usant l'insert.

Le cotyle de la présente invention remédie à ces inconvénients par le fait qu'il ne présente aucune vis pour sa fixation dans la masse osseuse qui le reçoit.

Un cotyle de prothèse selon le préamble de la revendication 1 est decrit dans le document FR-A-2 770 769.

A cet effet, l'invention a donc pour objet un cotyle de prothèse de hanche, à double mobilité, comprenant une cupule, un insert sphérique coopérant à rotation dans la cupule et comportant un logement central rétentif pour la tête d'une tige fémorale, la cupule ayant un bord tangent par un point à un plan diamétral du cotyle et s'étendant de part et d'autre de ce point au-delà de ce plan diamétral de manière à former une lèvre de rétention de l'insert à l'opposé du point susdit. Selon l'invention, la cupule étant en acier inoxydable, sa surface extérieure est pourvue d'une ceinture de cannelures croisées formant une bande adjacente auxdits bords et dont la largeur est plus importante du côté de la lèvre que du côté opposé tandis que la surface extérieure de cette cupule, y compris la ceinture cannelée, est traitée par un revêtement en titane poreux suivi d'un revêtement d'hydroxy-apatite.

On s'est rendu compte après de nombreuses expérimentations, que la présence de ces cannelures et le traitement de la surface convexe de la cupule formaient des moyens d'ancrage et de maintien de celle-ci dans la masse osseuse qui étaient au moins équivalents aux vis utilisées jusqu'à ce jour. Sa mise en place dans la masse osseuse de l'os iliaque ne demande plus alors qu'un fraisage de celle-ci, l'orientation de la cupule étant le seul moyen nécessaire pour la dédier à une hanche gauche ou une hanche droite. Cette caractéristique de l'invention permet d'éviter des implantations défectueuses d'une cupule par exemple destinée à une hanche gauche et implantée dans une hanche droite, les vis ou au moins l'une d'elles pouvant être malencontreusement implantées dans des parois extrêmement minces de l'os iliaque alors qu'elles étaient destinées à pénétrer dans une masse osseuse importante. Ce défaut d'implantation peut conduire à un affaiblissement de l'os iliaque qui lui-même pourrait entraîner des complications post-opératoires importantes que l'invention permet d'éviter. Cette caractéristique permet également de préserver l'intégrité de la surface de glissement de l'insert dans la cupule.

Dans une variante préférée de réalisation de l'invention, la cupule comprend en une seule pièce un pion extérieur sensiblement radial situé sur le méridien passant par le point médian de la lèvre de rétention et entre 30 et 60° de latitude par rapport à ce point. Ce pion se loge dans un perçage réalisé au fond du chambrage ménagé dans l'os iliaque par le chirurgien, perçage dont on est sûr qu'il concerne une masse osseuse importante. Le pion constitue un élément de stabilisation de la cupule dans l'os s'opposant à son descellement si la prothèse subit un effort tendant à sa luxation. La surface extérieure de ce pion est bien entendu traitée de la même manière que le reste de la cupule.

D'autres caractéristiques et avantages de l'invention ressortiront de la description donnée ci-après d'un de ses modes de réalisation.

Il sera fait référence aux dessins annexés parmi lesquels :
- la figure 1 est une vue générale en perspective d'une prothèse de hanche complète mettant en oeuvre un cotyle selon l'invention,
- la figure 2 illustre par une vue extérieure la cupule du cotyle selon l'invention,
- la figure 3 illustre un insert du cotyle selon l'invention destiné à coopérer avec la cupule de la figure 2,
- les figures 4 et 5 illustrent par des vues en coupe la position relative d'une tête de prothèse avec un cotyle selon l'invention, selon que la tête de prothèse est de petit diamètre (figure 4) ou de grand diamètre (figure 5),
- la figure 6 illustre par une vue extérieure une cupule du cotyle selon l'invention équipée de son pion de retenue,
- la figure 7 illustre par une vue en coupe la géométrie de l'insert du cotyle selon l'invention retardant son usure et la perte de son pouvoir rétentif.

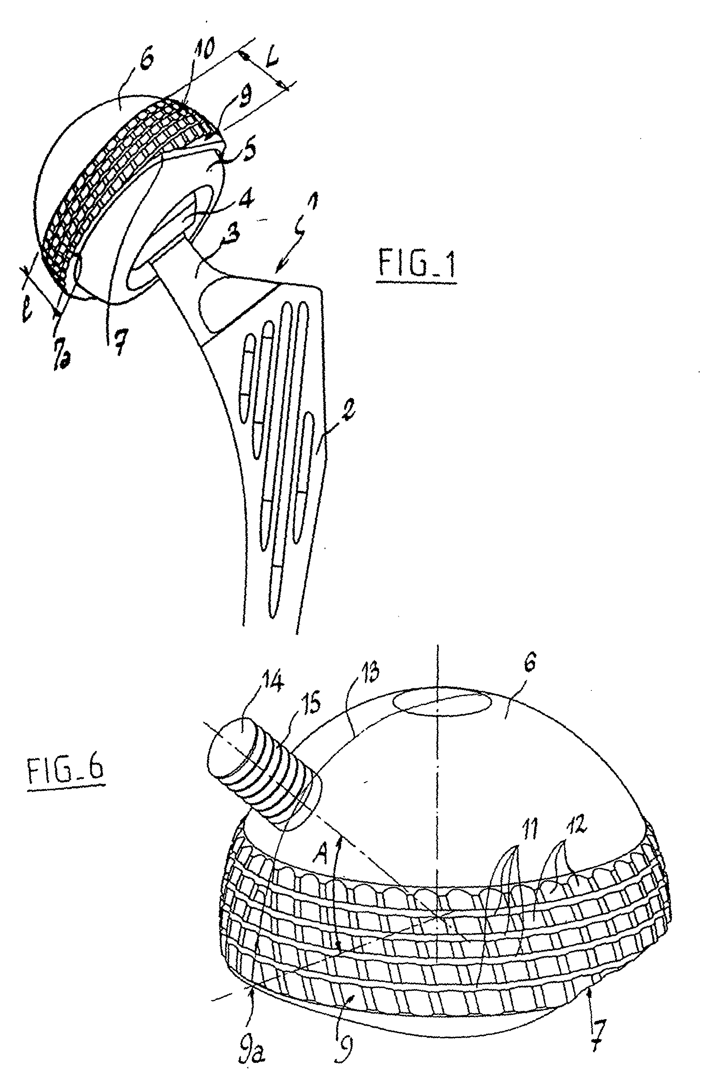

La prothèse de hanche représentée à la figure 1 comprend une tige 1 formée d'une partie 2 d'implantation dans le fémur, d'un col 3 et d'une tête sphérique 4 montée de manière connue à l'extrémité du col 3.

La tête sphérique 4 est logée de manière rétentive à l'intérieur d'un insert 5 lui-même monté à rotation sphérique à l'intérieur d'une cupule 6. La tête 4 est en général en céramique, l'insert 5 est en polyéthylène et la cupule 6 est en acier inoxydable (par exemple Z6CNU). En effet on sait que les qualités de frottement entre l'acier inoxydable et le polyéthylène sont parmi les meilleures aujourd'hui réalisées. L'inconvénient de l'acier inoxydable réside dans son faible pouvoir d'ancrage dans une masse osseuse, ce qui a conduit jusqu'à ce jour à implanter des cupules en acier inoxydable à l'aide de vis d'ancrage.

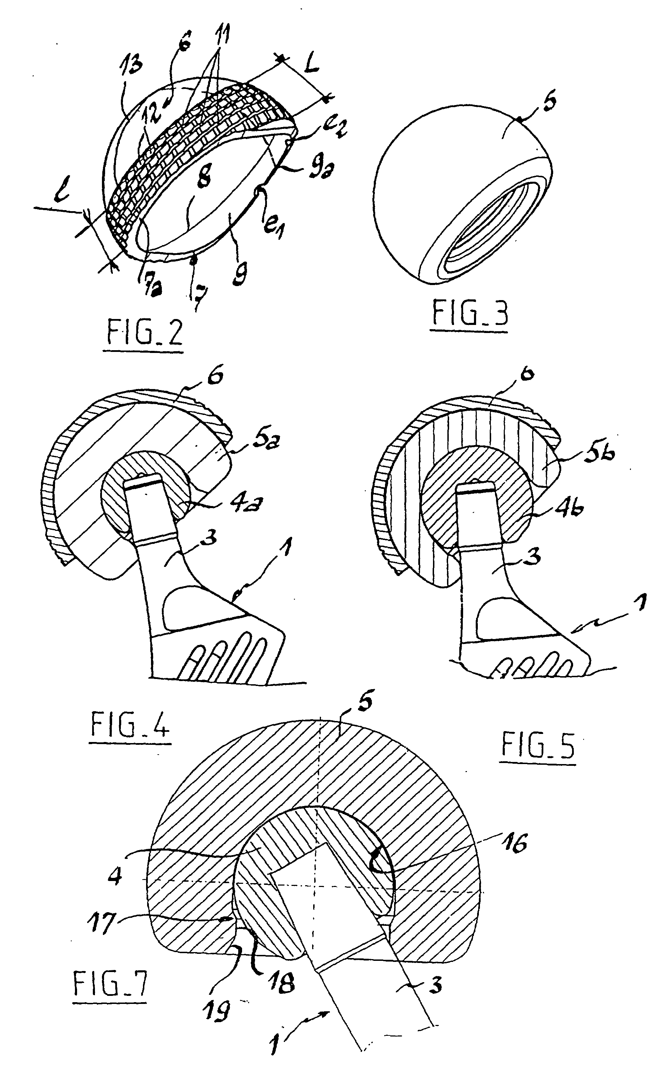

Ainsi que cela est plus clairement représenté à la figure 2, la cupule 6 possède un bord 7 dont un point 7a, avant sur le dessin, est contenue dans un plan diamétral de la cupule 6 dont la trace avec la surface sphérique intérieure de cette cupule est notée par la ligne 8. En dehors du point 7a et de chaque côté de celui-ci, le bord 7 s'étend au-delà de ce plan diamétral et définit ainsi sous ce plan, à l'opposé du sommet de la cupule 6, une lèvre de rétention 9 dont la surface interne prolonge la surface hémisphérique située au-dessus de la trace 8. Cette lèvre 9 forme un élément de retenue de l'insert 5 (figures 1 et 3) lorsque le débattement de la prothèse est tel que le col 3 de la tige vienne heurter ou prendre appui sur le bord 7a de la cupule.

La surface extérieure de la cupule 6 possède un réseau 10 de cannelures ou plus exactement une texturation formée dans une surépaisseur de la cupule en forme de ceinture formant une bande dont la largeur 1 au voisinage du point 7a est inférieure à la largeur L au voisinage du point médian 9a du bord de la lèvre de rétention 9. Cette texturation est en fait réalisée par des rainures circulaires 11 parallèles au plan équatorial contenant le point 7a du bord 7 coupé par des rainures 12 qui sont inclinées par rapport aux méridiens de la cupule.

On a noté en 13 le méridien qui passe par le point 7a et par le point médian 9a de la lèvre 9 à l'opposé du point 7a et on notera que l'inclinaison des rainures 12 est dans un sens d'un côté de ce méridien 13 et dans l'autre sens de l'autre côté de ce méridien. La surface extérieure de la cupule ainsi formée subit un double traitement. Il s'agit tout d'abord d'un dépôt de titane poreux par la technique des plasmas (ou toute technique équivalente : dépôt de vapeur...). Sur ce titane on applique ensuite une couche d'hydroxy-apatite. Le rôle du titane est d'une part de constituer une barrière à l'hydroxy-apatite qui aurait tendance à diffuser dans l'acier inoxydable et d'autre part de créer des micro-reliefs garnissant l'ancrage osseux surfacique.

On remarquera à la figure 2 la présence de deux encoches ou embrèvements E1, E2 qui servent au détrompage de la mise en place d'un outil d'impaction s'appuyant sur le bord 7 de-la cupule par un contour identique à celui de ce bord, ce qui permet de mettre en place la cupule dans l'os sans qu'il y ait contact entre la surface interne de cette cupule et l'outil d'impaction, contact qui tend inévitablement à altérer la qualité de surface intérieure de la cupule donc la qualité du frottement entre l'insert et cette dernière.

Les figures 4 et 5 illustrent le fait qu'à la figure 4 la tête 4a de la tige de prothèse 1 est de petit diamètre si bien que l'insert 5a est d'une épaisseur plus importante que l'insert 5b représenté à la figure 5 correspondant à une prothèse dans laquelle la tête 4b de la tige 1 est de diamètre plus grand. On notera en comparant ces figures que l'encombrement extérieur des inserts 5a et 5b est strictement identique. Ces inserts peuvent donc être produits à partir d'un même moule, seule l'opération de reprise consistant à ménager le logement rétentif diffère.

La cupule représentée en vue extérieure à la figure 6 contient des éléments déjà décrits en regard des figures précédentes avec les mêmes références. Cette cupule comporte, de manière préférée, un pion 14 orienté radialement par rapport à la surface extérieure de la cupule 6 et centré sur le méridien 13 déjà décrit qui relie le point 9a de la lèvre 9 du point 7a du bord 7 compris dans un plan diamétral de la cupule. Ce pion 14 est situé sur la partie du méridien voisine du point médian 9a de la lèvre 9 et à une latitude A par rapport à ce point comprise entre 30 et 60°. Le pion 14 est équipé d'une surface extérieure 15 formant des reliefs en dents de sapin.

Enfin à la figure 7 on a représenté l'insert 5 dans lequel est montée la sphère 4 d'une tige 1. Le logement rétentif 16 de l'insert possède une entrée 17 de diamètre plus petit que celui de la sphère 4, prolongé vers l'extérieur par un canal cylindrique 18 ouvert sur l'extérieur par un chanfrein conique 19. Le chanfrein 19 forme une butée au col 3 de la tige 1 lors des débattements de l'articulation qui empêche le contact de ce col avec l'entrée 17, préservant ainsi le pouvoir rétentif de l'insert. Cette disposition est avantageuse car elle prolonge la durée de vie de la prothèse comparativement aux prothèses à double mobilité connues qui ne possèdent pas cette prolongation cylindrique 18 et chanfreinée 19 de l'entrée du logement rétentif. Bien entendu, l'angle de débattement de la tige dans l'insert de l'invention est en conséquence légèrement diminué, alors qu'on le cherchait maximum dans les prothèses connues, sans inconvénient cependant pour le bon fonctionnement de la prothèse du fait de la mobilité de l'insert dans la cupule. En d'autres termes, l'avantage de cette géométrie est de donner à l'insert une masse de polyéthylène à user par la tige avant d'affecter le pouvoir rétentif.

## Revendications

1. Cotyle de prothèse de hanche, à double mobilité, comprenant une cupule (6), un insert (5) sphérique coopérant à rotation dans la cupule (6) et comportant un logement central rétentif pour la tête (4) d'une tige fémorale (1), la cupule (6) ayant un bord (7) tangent par un point (7a) à un plan diamétral du cotyle et s'étendant de part et d'autre de ce point au-delà de ce plan diamétral de manière à former une lèvre (9) rétentive de l'insert (5), à l'opposé du point (7a) susdit, **caractérisé en ce que**, la cupule étant en acier inoxydable, sa surface extérieure est pourvue d'une ceinture de cannelures (11, 12) croisées formant une bande (10) adjacente audit bord (7) et dont la largeur (L) est plus importante du côté de la lèvre (9) que du côté opposé et **en ce que** la surface extérieure de cette cupule (6), y compris la ceinture (10) cannelée, est traitée par un revêtement de titane poreux suivi d'un revêtement d'hydroxy-apatite.

2. Cotyle selon la revendication 1, **caractérisé en ce que** la cupule (6) comprend en une seule pièce un pion extérieur (14) sensiblement radial situé sur le méridien (13) passant par le point médian (9a) de la lèvre de rétention (9) et entre 30 et 60° de latitude par rapport à ce point.

3. Cotyle selon la revendication 2, **caractérisé en ce que** la surface extérieure (15) du pion (14) est pourvue de reliefs en dents de sapin.

4. Cotyle selon l'une de revendications précédentes, **caractérisé en ce que** le bord (7) de la cupule comporte au moins deux embrèvements (E1, E2) destinés au détrompage de la mise en place d'un outil d'impaction.

5. Cotyle selon l'une des revendications précédentes, **caractérisé en ce que** l'entrée étroite (17) du logement rétentif (16) de l'insert est prolongé vers l'extérieur d'un canal cylindrique (18) terminé par un chanfrein conique (19).

## Patentansprüche

1. Hüftprothesengelenkpfanne mit doppelter Mobilität, umfassend eine Kuppel (6), einen sphärischen Einsatz (5), der drehbar in der Kuppel (6) aufgenommen ist und eine zentrale Rastaufnahme für den Kopf (4) eines Oberschenkelschaftes (1) hat, wobei die Kuppel (6) einen Rand (7) hat, der in einem Punkt (7a) eine Durchmesserebene der Gelenkpfanne berührt und sich auf beiden Seiten dieses Punktes über diese Durchmesserebene hinaus erstreckt, derart, dass er gegenüber dem genannten Punkt (7a) eine Rastlippe (9) für den Einsatz (5) ausbildet, **dadurch gekennzeichnet, dass**, während die Kuppel aus rostfreiem Stahl ist, ihre Außenfläche mit einem Gürtel von sich kreuzenden Rillen (11, 12) versehen ist, der ein Band (10) bildet, das an den Rand (7) angrenzt und dessen Breite (L) auf der Seite der Lippe (9) größer ist als auf der gegenüberliegenden Seite, und dass die Außenfläche dieser Kuppel (6) einschließlich des geriffelten Gürtels (10) mit einer porösen Titanbeschichtung behandelt ist, auf die eine Hydroxyapatit-Beschichtung folgt.

2. Gelenkpfanne nach Anspruch 1, **dadurch gekennzeichnet, dass** die Kuppel (6) einen einstückig angeformten äußeren Zapfen (14) aufweist, der im wesentlichen radial ist und sich auf dem durch den mittleren Punkt (9a) der Rastlippe (9) verlaufenden Meridian (13) sowie zwischen dem 30. und 60. Breitengrad relativ zu diesem Punkt befindet.

3. Gelenkpfanne nach Anspruch 2, **dadurch gekennzeichnet, dass** die Außenfläche (15) des Zapfens (14) mit einem Sägezahnprofil versehen ist.

4. Gelenkpfanne nach einem der vorhergehenden Ansprüche, **dadurch gekennzeichnet, dass** der Rand (7) der Kuppel mindestens zwei Aussparungen (E1, E2) aufweist, die für ein irrtumsfreies Ansetzen eines Einsetzwerkzeuges bestimmt sind.

5. Gelenkpfanne nach einem der vorhergehenden Ansprüche, **dadurch gekennzeichnet, dass** die schmale Öffnung (17) der Rastaufnahme (16) des Einsatzes sich nach außen hin durch einen zylindrischen Kanal (18) fortsetzt, der mit einer konisch abgeschrägten Kante abschließt.

## Claims

1. An acetabular cup assembly with double mobility for a hip prosthesis, the assembly comprising a cup (6), a spherical insert (5) co-operating in rotation in the cup (6) and including a central housing for retaining the head (4) of a femoral stem (1), the cup (6) having an edge (7) that is tangential at a point (7a) to a diametral plane of the acetabular cup assembly and that extends on either side of said point beyond said diametral plane so as to form an insert-retaining lip (9) opposite from said point (7a), the cup assembly being **characterized in that**, the cup being of stainless steel, its outside surface is provided with a belt of crossed fluting (11, 12) forming a strip (10) adjacent to said edge (7) and of width (L) that is greater on the side of the lip (9) than on the opposite side, and **in that** the outside surface of said cup (6), including the fluted belt (10) is treated with a porous titanium coating followed by a hydroxyapatite coating.

2. An acetabular cup assembly according to claim 1, **characterized in that** the cup (6) is integral with a substantially radial external peg (14) situated on the meridian (13) passing through the medial point (9a) of the retaining lip (9) and at a latitude of 30° to 60° relative to said point.

3. An acetabular cup assembly according to claim 2, **characterized in that** the outside surface (15) of the peg (14) is provided with Christmas-tree-shaped serrations in relief.

4. An acetabular cup assembly according to any preceding claim, **characterized in that** the edge (7) of the cup includes at least two setbacks (E1, E2) for ensuring proper positioning of an impacting tool.

5. An acetabular cup assembly according to any preceding claim, **characterized in that** the narrow entry (17) of the insert-retaining housing (16) is extended outwards by a cylindrical channel (18) terminated by a conical chamfer (19).
